(19) Europäisches Patentamt
European Patent Office
Office européen des brevets

(11) EP 2 476 712 A1

(12) **EUROPÄISCHE PATENTANMELDUNG**

(43) Veröffentlichungstag:
**18.07.2012 Patentblatt 2012/29**

(21) Anmeldenummer: **11151299.2**

(22) Anmeldetag: **18.01.2011**

(51) Int Cl.:
***C08F 216/14*** *(2006.01)* ***C08F 230/02*** *(2006.01)*
***C08F 2/22*** *(2006.01)* ***C08F 2/24*** *(2006.01)*
***C08F 2/26*** *(2006.01)* ***C08F 2/30*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Cognis IP Management GmbH**
**40589 Düsseldorf (DE)**

(72) Erfinder:
- **Schliwka, Thomas**
  **51427 Bergisch-Gladbach (DE)**
- **Mausberg, Thomas**
  **42781 Haan (DE)**
- **Held, Uwe**
  **42553 Velbert (DE)**
- **Klagge, Ronald**
  **40699 Erkrath (DE)**
- **Busch, Stefan**
  **40597 Düsseldorf (DE)**
- **Scherer, Markus, Dr.**
  **50733 Köln (DE)**

(54) **Copolymerisierbare Tenside**

(57) Vorgeschlagen wird Verwendung von Verbindungen der Formel (I),

$$R\text{-}CH(O\text{-}(AO)_mX)\text{-}CH_2\text{-}O\text{-}(AO)_n\text{-}CH_2\text{-}CH{=}CH_2 \quad (I)$$

worin bedeuten:
- R einen Alkylrest mit 8 bis 18 C-Atomen, der gesättigt oder ungesättigt, geradkettig oder verzweigt sein kann,
- X eine Sulfat- oder Phosphatgruppe oder Wasserstoff,
- (AO) eine Alkylenoxideinheit, ausgewählt aus der Gruppe Ethylenoxid, Propylenoxid und Butylenoxid,
- n eine Zahl im Bereich von 0 bis 50 und
- m Null oder eine Zahl im Bereich von 1 bis 30

als copolymerisierbare Emulgatoren in der Emulsionspolymerisation olefinisch ungesättigter Monomerer.

EP 2 476 712 A1

## Beschreibung

### Gebiet der Erfindung

Die Erfindung liegt auf dem Polymersektor und betrifft Allylether spezieller Struktur sowie deren Verwendung als Emulgatoren in der Emulsionspolymerisation sowie ein Verfahren zur Herstellung von Polymeren durch Emulsionspolymerisation unter Verwendung der erfindungsgemäßen Allylether, die spezielle copolymerisierbare Emulgatoren sind.

### Stand der Technik

Bei der Emulsionspolymerisation handelt es sich um ein spezielles Verfahren der Polymerisation, bei dem gering wasserlösliche olefinisch ungesättigte Monomere mit Hilfe von Emulgatoren in Wasser emulgiert und unter Verwendung wasserlöslicher Initiatoren wie beispielsweise Kaliumperoxodisulfat oder Redoxinitiatoren polymerisiert werden. Anionische und/oder nicht-ionische Tenside sind hierbei die wesentliche Bestandteile. Über den Micellaufbau in der wässrigen Lösung gewährleisten sie den Prozess der Emulsionspolymerisation.

Copolymerisierbare Emulgatoren sind in der Industrie sehr gefragt, da sie in die wachsende Polymerkette vollständig oder teilweise eingebaut werden und dadurch beispielsweise die Migration freier Emulgatormoleküle im Endanwendungsprodukt herabsetzen. Copolymerisierbare Emulgatoren nehmen eine Mittelstellung zwischen Monomeren und herkömmlichen Emulgatoren ein. Hierbei müssen sie in Bezug auf ihre Reaktivität auf das verwendete Monomersystem abgestimmt sein und dürfen die Eigenschaften des entstehenden Polymers nicht negativ verändern. Gleichzeitig dürfen sie durch das Vorhandensein einer reaktiven Gruppe ihre emulgativen Eigenschaften nicht verlieren. Aufgrund dieser Kombination spezieller Eigenschaften besteht seitens der Industrie eine große Nachfrage an neuen copolymerisierberen Emulgatoren.

Die deutsche Offenlegungsschrift DE-A-10,340,081 beschreibt copolymerisierbare Tenside der Formel HOOC-CH=CH-COO-$(BO)_z(PO)y(EO)_xR^1$, in der $R^1$ für einen Alkylrest oder Alkylphenolrest mit 8 bis 24 Kohlenstoffatomen, BO für eine Butylenoxideinheit, PO für eine Propylenoxideinheit und EO für eine Ethylenoxideinheit steht und die Zahlen x, y und z unabhängig voneinander für 0 oder Zahlen von 1 bis 50 stehen, mit der Maßgabe, dass mindestens eine der Zahlen x, y und z von 0 verschieden ist, wobei die Carboxylgruppe teilweise oder ganz in neutralisierter Form vorliegen kann und die C=C-Doppelbindung cis- oder trans-konfiguriert sein kann.

EP-A-1,825,908 beschreibt spezielle Verbindungen der Struktur (A)

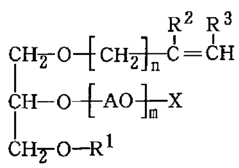

in der $R^1$ eine $C_{8-20}$ Kohlenwasserstoffgruppe, $R^2$ und $R^3$ Wasserstoff oder eine Methylgruppe, (AO) eine $C_{2-4}$ Alkylenoxidgruppe, X eine ionische hydrophile Gruppe, n eine Zahl von 0 bis 12 und m eine Zahl von 0 bis 100 bedeuten. Diese Verbindungen (A) werden in Kombination mit bestimmten Stickstoffverbindungen (B) eingesetzt, wobei der speziellen Zusammensetzung enthaltend (A) und (B) die Rolle eines Emulgators bei der Emulsionspolymerisation zukommt.

### Beschreibung der Erfindung

Der vorliegenden Erfindung lag die Aufgabe zugrunde, Verbindungen bereitzustellen, die sich allein oder in Abmischung mit anderen Verbindungen als copolymerisierbare Emulgatoren für die Emulsionspolymerisation eignen.

Bei der Verwendung als Emulgatoren für die Emulsionspolymerisation sollten diese insbesondere bewirken, dass nur eine geringe Koagulatbildung erfolgt. Weiterhin sollten diese copolymerisierbaren Emulgatoren in wässriger Angebotsform gießbar bzw. pumpbar sein.

Schließlich sollten durch die Verwendung als Emulgatoren bei der Emulsionspolymerisation Latices zugänglich sind, die gegenüber solchen Latices, die mit vergleichbaren nicht-copolymerisierbaren Emulgatoren hergestellt werden, verbesserte Eigenschaften in Bezug auf die Wasserbeständigkeit und Kratzfestigkeit von hieraus hergestellten Polymerfilmen aufweisen.

Gegenstand der Erfindung sind zunächst Verbindungen der allgemeinen Formel (I),

$$R\text{-}CH(O\text{-}(AO)_mX)\text{-}CH_2\text{-}O\text{-}(AO)_n\text{-}CH_2\text{-}CH{=}CH_2 \qquad (I)$$

worin bedeuten:

- R einen Alkylrest mit 8 bis 18 C-Atomen, der gesättigt oder ungesättigt, geradkettig oder verzweigt sein kann,
- X eine Sulfat- oder Phosphatgruppe oder Wasserstoff,
- (AO) eine Alkylenoxideinheit, ausgewählt aus der Gruppe Ethylenoxid, Propylenoxid und Butylenoxid,
- n eine Zahl im Bereich von 0 bis 50 und
- m Null oder eine Zahl im Bereich von 1 bis 30. Ein weiterer Gegenstand der Erfindung ist die Verwendung von Verbindungen der allgemeinen Formel (I),

$$R\text{-}CH(O\text{-}(AO)_mX)\text{-}CH_2\text{-}O\text{-}(AO)_n\text{-}CH_2\text{-}CH{=}CH_2 \qquad (I)$$

worin bedeuten:

- R einen Alkylrest mit 8 bis 18 C-Atomen, der gesättigt oder ungesättigt, geradkettig oder verzweigt sein kann,
- X eine Sulfat- oder Phosphatgruppe oder Wasserstoff,
- (AO) eine Alkylenoxideinheit, ausgewählt aus der Gruppe Ethylenoxid, Propylenoxid und Butylenoxid,
- n eine Zahl im Bereich von 0 bis 50 und
- m Null oder eine Zahl im Bereich von 1 bis 30.

als copolymerisierbare Emulgatoren in der Emulsionspolymerisation olefinisch ungesättigter Monomerer. Dabei können im Zuge der Emulsionspolymeristion die Verbindungen (I) einzeln oder im Gemisch untereinander - gewünschtenfalls auch in Kombination mit herkömmlichen Emulgatoren - eingesetzt werden.

In der Formel (I) bedeutet der Baustein (AO) eine Alkylenoxideinheit, ausgewählt aus der Gruppe Ethylenoxid, Propylenoxid und Butylenoxid. Die Indices n und m bedeuten in der Formel (I) im Zusammenhang mit dem Strukturbaustein $(AO)_n$ und $(AO)_m$ die mittlere Anzahl der Alkylenoxid-Bausteine.

Die Verbindungen (I) können nach allen dem Fachmann bekannten Methoden hergestellt werden. Üblicherweise geschieht dies dadurch, dass man alpha-Olefin-Epoxide mit Anlagerungsprodukten von Ethylenoxid, Propylenoxid und/oder Butylenoxid an Allylalkohol unter Öffnung des Oxiranringes der Epoxide umsetzt. Die bei der Ringöffnung entstehende OH-Gruppe kann gewünschtenfalls in eine Sulfat- oder Phospatgruppe überführt werden; dabei kann vor der Überführung in eine Sulfat- oder Phospatgruppe gewünschtenfalls eine Alkoxylierung durchgeführt werden.

Anlagerungsprodukte von Ethylenoxid, Propylenoxid und/oder Butylenoxid an Allylalkohol sind wie dem Fachmann bekannt durch Umsetzung von Allylalkohol in Gegenwart eines Alkoxylierungs-Katalysators mit Ethylenoxid, Propylenoxid und/oder Butylenoxid zugänglich; dies sei durch das folgende Formelschema verdeutlich, in der (AO) und n dieselbe Bedeutung haben wie in der Formel (I): $CH_2{=}CH\text{-}CH_2\text{-}OH + n\ (AO) \rightarrow CH_2{=}CH\text{-}CH_2\text{-}O\text{-}(AO)_n\text{-}H$

Bei der Alkoxylierug kann man Ethylenoxid, Propylenoxid und Butylenoxid einzeln oder im Gemisch untereinander einsetzen. Im Hinblick auf den Index n sei in diesem Zusammenhang festgestellt, dass es sich hier um einen (statistischen) Mittelwert handelt; so bedeutet für $CH_2{=}CH\text{-}CH_2\text{-}O\text{-}(AO)_n\text{-}H$ die Aussage n = 1, dass man 1 mol Allylalkohol 1 mol Alkylenenoxid umgesetzt hat, x = 2 bedeutet, dass man 1 mol Allylalkohol mit 2 mol Alkylenoxid umgesetzt hat, x = 6 bedeutet, dass man 1 mol Allylalkohol mit 6 mol Alkylenoxid umgesetzt hat, usw. Mithin repräsentiert der Index n das molare Umsetzungsverhältnis von Allylakohol und eingesetztem Alkylenoxid, wobei die Allylalkoholalkoxylate sich je nach eingesetztem Katalysator in der Homologenverteilung unterscheiden können.

Der Index n bedeutet in der Formel (I) wie bereits ausgeführt eine Zahl im Bereich von 0 bis 50. Bevorzugt ist dabei 1 bis 30 und insbesondere 3 bis 10. Da n die Zahl der Alkylenoxideinheiten $(AO)_n$ bezeichnet und die Alkylenoxideinheiten ausgewählt werden aus der Gruppe Ethylenoxid, Propylenoxid und Butylenoxid sei ausdrücklich festgestellt, dass dies so zu verstehen ist, dass n die Summe aller im Gesamt-$(AO)_n$-Segment (worunter die durch $(AO)_n$ repräsentierte Gesamtheit verstanden wird) vorhandenen Ethylenoxid-, Propylenoxid- und Butylenoxid-Einheiten darstellt. Das Gesamt-$(AO)_n$-Segment kann auschließlich aus Ethylenoxid-, Propylenoxid- oder Butylenoxid-Einheiten aufgebaut sein oder diese Einheiten in gemischter Form - statistisch verteilt oder in Blockform - enthalten. Vorzugsweise ist das Gesamt-$(AO)_n$-Segment der Verbindungen (I) ausschließlich aus Ethylenoxid- und/oder Propylenoxid-Einheiten aufgebaut. Insbesondere ist das Gesamt-$(AO)_n$-Segment der Verbindungen (I) ausschließlich aus Ethylenoxid-Einheiten aufgebaut.

Der Index m bedeutet in der Formel (I) wie bereits ausgeführt Null oder eine Zahl im Bereich von 1 bis 30. In einer Ausführungsform ist m gleich Null. In einer weiteren Ausführungsform ist m im Bereich 1 bis 10 und insbesondere 3 bis 10. Da m die Zahl der Alkylenoxideinheiten $(AO)_m$ bezeichnet und die Alkylenoxideinheiten ausgewählt werden aus der Gruppe Ethylenoxid, Propylenoxid und Butylenoxid sei ausdrücklich festgestellt, dass dies so zu verstehen ist, dass m die Summe aller im Gesamt-$(AO)_m$ Segment (worunter die durch $(AO)_m$ repräsentierte Gesamtheit verstanden wird) vorhandenen Ethylenoxid-, Propylenoxid- und Butylenoxid-Einheiten darstellt. Das Gesamt-$(AO)_m$-Segment kann auschließlich aus Ethylenoxid-, Propylenoxid- oder Butylenoxid-Einheiten aufgebaut sein oder diese Einheiten in gemischter Form - statistisch verteilt oder in Blockform - enthalten. Vorzugsweise ist das Gesamt-$(AO)_m$-Segment der Verbindungen (I) ausschließlich aus Ethylenoxid- und/oder Propylenoxid-Einheiten aufgebaut. Insbesondere ist das Gesamt-$(AO)_m$-Segment der Verbindungen (I) ausschließlich aus Ethylenoxid-Einheiten aufgebaut.

Bei der Gruppe X handelt es sich um eine Sulfat- oder Phosphatgruppe oder um Wasserstoff.

In einer Ausführungsform handelt es sich bei der Gruppe X um Wasserstoff.

In einer Ausführungsform handelt es sich bei der Gruppe X um eine Sulfat- oder Phosphatgruppe. Dabei liegen die Sulfat- bzw. Phosphatgruppen X in den Verbindungen (I) in teilweise oder vollständig neutralisierter Form vor. Die Neutralisation der Sulfat- bzw. Phosphatgruppe kann beispielsweise mit Alkali- oder Erdalkalihydroxiden wie Natrium-, Kalium-, Calcium- oder Magnesiumhydroxid oder mit Aminen wie Ammoniak oder Ethanolaminen geschehen. Die Salzform der Verbindungen (I) zeichnet sich durch eine gute Wasserlöslichkeit aus.

Die Verbindungen der Formel (I) sind beispielsweise dadurch zugänglich, daß man einen alkoxylierten Allylalkohol mit einem alpha-Olefinoxid umsetzt und das erhaltene Produkt (mit X = H) gewünschtenfalls sulfatiert oder phosphatiert.

Die erfindungsgemäß einzusetzenden Verbindungen (I) lassen sich leicht und vollständig zusammen mit anderen, davon verschiedenen olefinisch ungesättigten Monomeren polymerisieren, wobei sie die Bildung einer schaumfreien und homogenen Emulsion fördern.

Diejenigen Verbindungen (I) bei denen X die Bedeutung einer Sulfat oder Phosphatgruppe hat sind bevorzugt. Sie werden im Rahmen der Emulsionspolymerisation vorzugsweise in teilweiser oder vollständig neutralisierter Form eingesetzt ("Salzform" der Sulfat- bzw. Phosphatgruppe). Diese ist leicht zugänglich, indem man die Verbindungen (I) nach üblichen Methoden teilweise oder ganz neutralisiert, beispielsweise mit Alkali- oder Erdalkalihydroxiden wie Natrium-, Kalium-, Calcium- oder Magnesiumhydroxid oder mit Aminen wie Ammoniak oder Ethanolaminen. Die Salzform der Verbindungen (I) zeichnet sich durch eine gute Wasserlöslichkeit aus.

Ein weiterer Gegenstand der Erfindung ist ein Verfahren zur Herstellung von Polymeren durch Emulsionspolymerisation olefinisch ungesättigter Monomere, bei dem man als copolymeriserbare Emulgatoren die oben genannten Verbindungen (I) einsetzt.

Das erfindungsgemäße Verfahren unter Einsatz der Verbindungen (I), insbesondere in Salzform, zeichnet sich dadurch aus, dass Polymere mit besonderer Scher- und Elektrolytbeständigkeit sowie niedrigem Koagulatgehalt erhalten werden.

In einer Ausführungsform der Erfindung werden Latices erhalten, die sich ihrerseits zusätzlich durch Stabilität gegenüber Temperaturschwankungen auszeichnen und bei denen keine Migration des Emulgators im Film festzustellen ist.

Ein weiterer Vorteil des erfindungsgemäßen Verfahrens besteht ferner darin, dass es praktisch schaumfrei ist und die Bildung flüchtiger organischer Stoffe zuverlässig vermieden wird. Da die Emulgatoren (I) praktisch quantitativ in das Polymer eingebaut werden, sind mit deren Verwendung auch keine Probleme hinsichtlich der biologischen Abbaubarkeit verbunden. Die olefinisch ungesättigten Verbindungen (I zeigen des weiteren praktisch keine Tendenz zur Homopolymerisation.

Es wurde gefunden, dass durch die Verwendung der Verbindungen (I) als Emulgatoren bei der Emulsionspolymerisation Latices zugänglich sind, die gegenüber solchen Latices, die mit vergleichbaren nicht-copolymerisierbaren Emulgatoren hergestellt wurden, verbesserte Eigenschaften in Bezug auf die Wasserbeständigkeit und Kratzfestigkeit von hieraus hergestellten Polymerfilmen aufweisen. Auch Kombinationen von der Verbindungen (I) mit (von (I) verschiedenen) Tensiden nicht-ionischer und anionischer Natur sind einsatzfähig und zeigen ebenfalls ein positives Eigenschaftsprofil.

Die erfindungsgemäßen Latices können beispielsweise in der Beschichtungsindustrie eingesetzt werden. Es wurde gefunden, daß Beschichtungen, die mit den erfindungsgemäßen Latices hergestellt wurden, einen höheren Korrosionsschutz gegenüber herkömmlichen Beschichtungen besitzen.

Weiterhin wurde gefunden, daß insbesondere Latices, die unter Einsatz von Verbindungen der allgemeinen Formel (I) als Emulgatoren hergestellt wurden, gegenüber solchen Latices, die mit herkömmlichen Emulgatoren hergestellt wurden, eine verbesserte Gefrier-Tau-Stabilität aufweisen.

<u>Monomere</u>

Die erfindungsgemäß einzusetzenden olefinisch ungesättigten Ester der allgemeinen Formel (I) eignen sich als Emulgatoren bei der Emulsionspolymerisation von nahezu sämtlichen technisch wichtigen, im wesentlichen wasserunlöslichen Monomeren, vorzugsweise aber (Meth)acryl-, Styrol- und Vinylverbindungen.

Typische Beispiele für diese Monomeren sind Vinylaromaten, z.B. Styrol, Divinylbenzol oder Vinyltoluol, polymerisierbare Olefine und Diolefine wie Propen, Butadien oder Isopren, Ester der Acryl- oder Methacrylsäure mit linearen oder verzweigten Alkoholen mit 1 bis 18 Kohlenstoffatomen, insbesondere von Alkoholen mit 1 bis 8 Kohlenstoffatomen und - besonders bevorzugt - von Methylestern, Ethylestern und Butylestern derselben, Vinylester von Säuren mit 2 bis

12 Kohlenstoffatomen, insbesondere Vinylacetat, Vinylpropionat, Vinyl-2-ethylhexanat und Vinyllaurat, Vinylalkylether mit 1 bis 8 Kohlenstoffatomen aufweisenden Alkylgruppen, Vinylchlorid, Vinylidenchlorid und dergleichen.

Monomere, die ausgewählt sind auf die Gruppe der Alkylacrylate, Styrolacrylate VeoVa-Verbindungen oder Mischungen hieraus, mit oder ohne Zusatz von Acrylsäure oder Methacrylsäure, sind im Rahmen der vorliegenden Erfindung besonders bevorzugt.

Die Monomeren können in Gegenwart der erfindungsgemäß einzusetzenden copolymerisierbaren Emulgatoren (I) homopolymerisiert oder mit anderen der genannten Verbindungen aus der vorstehenden Aufzählung copolymerisiert werden. Weiterhin können Copolymerisationen durchgeführt werden, bei denen bis zu 50 Gew.-% weitere, von den erfindungsgemäßen Verbindungen (I) verschiedene, an sich teilweise oder vollständig wasserlösliche Monomere beteiligt sind, zum Beispiel Acrylnitril, Methacrylnitril, Halbester der Malein- bzw. Fumarsäure mit 1 bis 8 Kohlenstoffatomen, Acrylsäure, Methacrylsäure, Maleinsäure, Fumarsäure, Crotonsäure und/oder Itaconsäure.

In einer Ausführungsform setzt man in dem erfindungsgemäßen Verfahren als Monomere Kombinationen von Styrol/Butylacrylat, Vinylacetat/Butylacrylat oder Styrol/Butadien ein.

Co-Emulgatoren

Weiterhin ist es auch möglich, die erfindungsgemäß einzusetzenden Verbindungen (I) in Kombination mit bekannten nichtionischen und/oder anionischen Co-Emulgatoren einzusetzen. Dies kann zu Dispersionen mit erhöhter Stabilität, z.B. gegenüber Scherkräften, Temperatureinflüssen und Elektrolyten führen. Die Co-Emulgatoren werden dabei in Mengen von 0,5 bis 5, vorzugsweise 1 bis 3 Gew.-%, bezogen auf die Gesamtheit der eingesetzten Monomere, zugegeben. Dabei ist es möglich, die Co-Emulgatoren zu Beginn der Polymerisation zusammen mit den Emulgatoren vorzulegen oder sie im Verlauf der Polymerisation zuzudosieren. Eine weitere Variante sieht vor, eine Präemulsion unter Verwendung oder Mitverwendung der Co-Emulgatoren herzustellen und diese im Verlauf der Polymerisation zuzudosieren. Es ist auch möglich, zur Nachstabilisierung der unter Verwendung der erfindungsgemäßen Acrylsäure- und/oder Methacrylsäureester erhaltenen Dispersionen diese mit Co-Emulgatoren zu versetzen.

Die erfindungsgemäß einzusetzenden Verbindungen (I) können auch zusammen mit Schutzkolloiden eingesetzt werden. Typische Beispiele für derartige Schutzkolloide sind vollständig oder teilweise verseifte Homo- und/oder Copolymere des Vinylacetats, z.B. teilverseiftes Polyvinylacetat, oder vollständig verseifte Copolymere aus Vinylacetat und Vinylethern. Bevorzugte Copolymere weisen 1 bis 4 Kohlenstoffatome im Etherteil des Polyvinylethers auf. Weitere Schutzkolloide können von Polysacchariden abgeleitet sein. So sind insbesondere Cellusoseether wie Hydroxyethylcellulose, Hydroxypropylcellulose, Carboxymethylcellulose, Methylcellulose, Ethylcellulose oder Cellulose-Mischether geeignet. Weiterhin geeignet sind Polyacrylamid sowie dessen Copolymere mit Acrylsäure, Acrylnitril oder Acrylestern. Auch Kondensationsprodukte aus Naphthalinsulfonsäure und Formaldehyd oder andere wasserlösliche Formaldehydharze, insbesondere Harnstoff-Formaldehyd-Harze, können verwendet werden. Schließlich sind Casein, Gelatine, Gummi arabicum sowie natürliche Stärke und substituierte Stärkederivate wie Hydroxyethylstärke geeignete Schutzkolloide.

Emulsionspolymerisation

In einer Ausführungsform setzt man die Emulgatoren (I) bei der Emulsionspolymerisation in Mengen von insgesamt 0,1 bis 25 Gew.-% - bezogen auf die Summe der Monomere - ein.

Die unter Verwendung der Verbindungen (I) im ersten Schritt des Verfahrens üblicherweise herzustellenden wäßrigen Dispersionen weisen in der Praxis 15 bis 75 Gew.-% polymerisierte Monomere (Trockenrückstand) in Wasser oder einem Gemisch von Wasser und wasserlöslichen organischen Lösemitteln auf. Bevorzugt ist der Bereich von 20 bis 60 Gew.-% Trockenrückstand; jedoch sind für spezielle Anwendungen auch wäßrige Dispersionen mit weniger als 15 Gew.-% Trockenrückstand herstellbar. Bei den vorgenannten Verfahren zur Emulsionspolymerisation können auch weitere übliche Polymerisationshilfsstoffe eingesetzt werden, insbesondere Initiatoren, z.B. anorganische Peroxidverbindungen wie Kalium- oder Ammoniumpersulfat oder Wasserstoffperoxid; weiterhin organische Peroxidverbindungen oder organische Azoverbindungen, soweit diese für die Emulsionspolymerisation verwendbar sind. Die Initiatoren werden in üblichen Mengen, d.h. von 0,05 bis 2 Gew.-%, vorzugsweise von 0,1 bis 0,5 Gew.-% eingesetzt. Weitere geeignete Hilfsstoffe sind Puffersubstanzen, z.B. Natriumhydrogencarbonat, Natriumpyrophosphat oder Natriumacetat, die in Mengen von bis zu 2 Gew.-% eingesetzt werden können. Auch Beschleuniger wie Formaldehydsulfoxylat können eingesetzt werden. Weiterhin können übliche, bei der Emulsionspolymerisation verwendete Molekulargewichtsregler, z.B. Butenol oder auch organische Thioverbindungen wie Mercaptoethanol, Thioglycolsäure, Octylmercaptan oder tert.-Dodecylmercaptan verwendet werden. Für die Durchführung der Polymerisationsverfahren kommen verschiedene, üblicherweise bei der Emulsionspolymerisation angewandte Methoden in Betracht, z.B. eine Gesamtvorlage aller Reaktanden, ein Monomerenzulauf oder ein Emulsionszulauf. Im Allgemeinen wird dazu die Temperatur des Polymerisationsmediums in einem Bereich von 40 bis 100, insbesondere 50 bis 90°C, gehalten. Als pH-Wert wird zweckmäßigerweise ein Bereich zwischen 3 und 9 eingehalten, jedoch ist mit den erfindungsgemäßen Verbindungen auch eine Emulsionspolymerisation

bei niedrigeren pH-Werten möglich. Die vorgenannten möglichen Verfahrensvarianten zur Emulsionspolymerisation werden zweckmäßigerweise in kühl- und heizbaren, mit Rührer und Temperaturmeßeinrichtung versehenen Behältern, z.B. in Rührdruckkesseln, durchgeführt. Ebenfalls möglich ist die Verwendung von Rohrschlangenreaktoren oder so genannten Loop-Reaktoren. Nach Beendigung der Polymerisation wird die Polymerisatdispersion zweckmäßigerweise abgekühlt und über Siebeinrichtungen aus dem Reaktor entfernt. Falls die Reaktionsprodukte als Festprodukte isoliert werden sollten, wird die Polymerisatdispersion zweckmäßigerweise ausgefällt oder sprühgetrocknet. Bevorzugt ist jedoch eine direkte Verwendung der bei der Polymerisation erhaltenen Dispersionen als Bindemittel für Farben, Klebstoffe, Papierstreichmassen und andere Beschichtungsmittel. Weitere Bedingungen für Verfahren zur Emulsionspolymerisation unter Verwendung der erfindungsgemäß einzusetzenden Verbindungen (I) können von dem Fachmann in üblicher Weise an die jeweiligen Erfordernisse angepasst oder frei gewählt werden.

**Patentansprüche**

**1.** Verbindungen der Formel (I),
$R\text{-}CH(O\text{-}(AO)_mX)\text{-}CH_2\text{-}O\text{-}(AO)_n\text{-}CH_2\text{-}CH{=}CH_2$ (I)
worin bedeuten:

- R einen Alkylrest mit 8 bis 18 C-Atomen, der gesättigt oder ungesättigt, geradkettig oder verzweigt sein kann,
- X eine Sulfat- oder Phosphatgruppe oder Wasserstoff,
- (AO) eine Alkylenoxideinheit, ausgewählt aus der Gruppe Ethylenoxid, Propylenoxid und Butylenoxid,
- n eine Zahl im Bereich von 0 bis 50 und
- m Null oder eine Zahl im Bereich von 1 bis 30.

**2.** Verwendung von Verbindungen der Formel (I),
$R\text{-}CH(O\text{-}(AO)_mX)\text{-}CH_2\text{-}O\text{-}(AO)_n\text{-}CH_2\text{-}CH{=}CH_2$ (I)
worin bedeuten:

- R einen Alkylrest mit 8 bis 18 C-Atomen, der gesättigt oder ungesättigt, geradkettig oder verzweigt sein kann,
- X eine Sulfat- oder Phosphatgruppe oder Wasserstoff,
- (AO) eine Alkylenoxideinheit, ausgewählt aus der Gruppe Ethylenoxid, Propylenoxid und Butylenoxid,
- n eine Zahl im Bereich von 0 bis 50 und
- m Null oder eine Zahl im Bereich von 1 bis 30,

als copolymerisierbare Emulgatoren in der Emulsionspolymerisation olefinisch ungesättigter Monomerer.

**3.** Verwendung gemäß Anspruch 2, wobei X eine Sulfat- oder Phosphatgruppe bedeutet.

**4.** Verwendung gemäß Anspruch 3, wobei die Sulfat- bzw. Phosphatgruppen in teilweise oder vollständig neutralisierter Form vorliegen.

**5.** Verwendung gemäß einem der Ansprüche 2 bis 4, wobei dere Alkoxylierungsgrad n im Bereich von 1 bis 30 liegt.

**6.** Verwendung gemäß einem der Ansprüche 2 bis 4, wobei dere Alkoxylierungsgrad n im Bereich von 3 bis 10 liegt und (AO) eine Ethylenoxid-Einheit bedeutet.

**7.** Verfahren zur Herstellung von Polymeren durch Emulsionspolymerisation olefinisch ungesättigter Monomere, bei dem man als copolymerisierbare Emulgatoren die Verbindungen (I) gemäß Anspruch 1 einsetzt.

Europäisches Patentamt
European Patent Office
Office européen des brevets

# EUROPÄISCHER RECHERCHENBERICHT

Nummer der Anmeldung
EP 11 15 1299

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | DE 102 39 932 A1 (CLARIANT GMBH [DE] CLARIANT PRODUKTE DEUTSCHLAND [DE]) 6. Mai 2004 (2004-05-06) * Absatz [0013]; Ansprüche * | 1-7 | INV. C08F216/14 C08F230/02 C08F2/22 C08F2/24 C08F2/26 C08F2/30 |
| A | EP 0 244 841 A2 (DAI ICHI KOGYO SEIYAKU CO LTD [JP]) 11. November 1987 (1987-11-11) * Seite 1, Zeile 3 - Seite 2, Zeile 15; Ansprüche * | 1-7 | |
| A | EP 1 318 157 A1 (KAO CORP [JP]) 11. Juni 2003 (2003-06-11) * Absatz [0001] - Absatz [0012]; Ansprüche * | 1-7 | |
| A | WO 2010/026065 A1 (BASF SE [DE]; HAYES PETER C [US]; HORWITZ RONAL J [US]) 11. März 2010 (2010-03-11) * Seite 2, Zeile 20 - Zeile 30; Ansprüche * | 1-7 | RECHERCHIERTE SACHGEBIETE (IPC) |
| A | US 5 371 159 A (TSUZUKI MASAHIDE [JP] ET AL) 6. Dezember 1994 (1994-12-06) * Spalte 2, Zeile 25 - Spalte 3, Zeile 21 * | 1-7 | C08F |

Der vorliegende Recherchenbericht wurde für alle Patentansprüche erstellt

1

| Recherchenort | Abschlußdatum der Recherche | Prüfer |
|---|---|---|
| Den Haag | 4. Juli 2011 | Grittern, Albert |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 11 15 1299

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-07-2011

| Im Recherchenbericht angeführtes Patentdokument | Datum der Veröffentlichung | Mitglied(er) der Patentfamilie | Datum der Veröffentlichung |
|---|---|---|---|
| DE 10239932 A1 | 06-05-2004 | US 2008227917 A1<br>US 2010029842 A1 | 18-09-2008<br>04-02-2010 |
| EP 0244841 A2 | 11-11-1987 | DE 3777441 D1<br>US 4814514 A<br>US 4939283 A | 23-04-1992<br>21-03-1989<br>03-07-1990 |
| EP 1318157 A1 | 11-06-2003 | DE 60117571 T2<br>WO 0222692 A1<br>US 2004048963 A1 | 18-01-2007<br>21-03-2002<br>11-03-2004 |
| WO 2010026065 A1 | 11-03-2010 | US 2010062264 A1 | 11-03-2010 |
| US 5371159 A | 06-12-1994 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10340081 A
- EP 1825908 A